Europäisches Patentamt

European Patent Office

Office européen des brevets

(11) Veröffentlichungsnummer: **0 286 170**
**B1**

(12) # EUROPÄISCHE PATENTSCHRIFT

(45) Veröffentlichungstag der Patentschrift:
**12.09.90**

(21) Anmeldenummer: **88200599.4**

(22) Anmeldetag: **30.03.88**

(51) Int. Cl.⁵: **A61B 17/22**

(54) **Lithotripsie-Arbeitsplatz.**

(30) Priorität: **04.04.87 DE 3711404**
**18.07.87 DE 3723920**

(43) Veröffentlichungstag der Anmeldung:
**12.10.88 Patentblatt 88/41**

(45) Bekanntmachung des Hinweises auf die Patenterteilung:
**12.09.90 Patentblatt 90/37**

(84) Benannte Vertragsstaaten:
**DE FR GB**

(56) Entgegenhaltungen:
**EP-A- 0 205 878**
**DE-U- 8 528 785**

(73) Patentinhaber: **Philips Patentverwaltung GmbH,**
**Wendenstrasse 35 Postfach 10 51 49,**
**D-2000 Hamburg 1(DE)**

(84) Benannte Vertragsstaaten: **DE**

(73) Patentinhaber: **N.V. Philips' Gloeilampenfabrieken,**
**Groenewoudseweg 1, NL-5621 BA Eindhoven(NL)**

(84) Benannte Vertragsstaaten: **FR GB**

(72) Erfinder: **Schwleker, Horst, Trenknerweg 21,**
**D-2000 Hamburg 52(DE)**
Erfinder: **Elff, Manfred, Dr., Volksdorfer Weg 52b,**
**D-2000 Hamburg 65(DE)**
Erfinder: **Pfeiffer, Wilfried, Berliner Strasse 46,**
**D-2085 Quickborn(DE)**
Erfinder: **Christiansen, Dieter, Dorfstrasse 44,**
**D-2071 Schönberg(DE)**

(74) Vertreter: **Hartmann, Heinrich, Dipl.-Ing. et al, Philips**
**Patentverwaltung GmbH**
**Wendenstrasse 35 Postfach 10 51 49,**
**D-2000 Hamburg 1(DE)**

**Beschreibung**

Die Erfindung betrifft einen Lithotripsie-Arbeitsplatz mit einem Patientenlagerungstisch, einer einen Röntgenstrahler und einen darauf zentrierten Bildwandler umfassenden Röntgeneinrichtung zur Ortung von Konkrementen, die um eine erste zur Tischlängsrichtung senkrechte, horizontale Achse schwenkbar ist, die den Zentralstrahl der Röntgeneinrichtung in einem Schnittpunkt schneidet, und mit einem unterhalb der Tischplatte befindlichen Stoßwellenerzeuger, der um eine durch seinen Fokuspunkt verlaufende zweite Achse bewegbar ist, wobei die Röntgeneinrichtung und der Stoßwellenerzeuger relativ zueinander so anordbar sind, daß der Fokuspunkt und der Schnittpunkt zusammenfallen.

Ein solcher Arbeitsplatz ist aus der EP-A 205 878 bekannt. Die zweite Achse verläuft dabei parallel zur Tischlängsrichtung und – da der Patient sich auf der Tischplatte befindet – oberhalb der Tischplatte. Zur Bewegung des Stoßarms um diese zweite Achse ist bei dem bekannten Arbeitsplatz daher ein c-förmiger Bogen vorgesehen, der sich um die zweite Achse herum krümmt. Um die Gefahr einer Kollision dieses an einem verschiebbaren Wagen befestigten Bogens mit dem Patientenlagerungstisch zu vermeiden, ist die Länge des c-Bogens begrenzt und damit auch der Bogen, auf dem der Stoßwellenerzeuger bewegt werden kann (bis zu maximal 20° nach jeder Seite).

Weiterhin ist aus dem DE-GM 8 528 785 bereits ein Lithotripsie-Arbeitsplatz bekannt, der einen Patientenlagerungstisch, eine aus zwei Röntgenstrahlern und zwei Bildwandlern bestehende Röntgeneinrichtung zur Ortung von Nierensteinen und zwei in Richtung ihres Fokuspunktes verschiebbare Stoßwellenerzeuger umfaßt, die sich unterhalb der Tischplatte des Patientenlagerungstisches befinden. Die beiden Stoßwellenerzeuger sind dabei jeweils einer Niere zugeordnet.

Es ist Aufgabe der vorliegenden Erfindung, einen Lithotripsie-Arbeitsplatz der eingangs genannten Art zu schaffen, der mit nur einem Stoßwellenerzeuger auskommt und der zur Bewegung des Stoßwellenerzeugers um die zweite Achse keinen c-förmigen Rahmen benötigt.

Diese Aufgabe wird erfindungsgemäß dadurch gelöst, daß die zweite Achse schräg verläuft und mit der Tischlängsrichtung eine vertikale Ebene definiert und daß der Stoßwellenerzeuger an einem Schwenkarm befestigt ist, der an einem unterhalb der Tischplatte befindlichen Lagerhalter um die zweite Achse schwenkbar angelenkt ist.

Da die Röntgeneinrichtung um die erste Achse schwenkbar ist, kann der Patient aus zwei verschiedenen Richtungen durchstrahlt werden, was die Ortung eines Nierensteins und seine Positionierung im Zentrum des Stoßwellenerzeugers mit nur einem Röntgenstrahler und nur einem Bildwandler ermöglicht. Wenn der Nierenstein in dem Schnittpunkt positioniert ist, kann der Stoßwellenerzeuger in die für die Steinzertrümmerung optimale Lage geschwenkt werden, wobei eine Schwenkung sowohl auf die linke als auch auf die rechte Seite des Patienten möglich ist, so daß mit nur einem Stoßwellenerzeuger Nierensteine sowohl in der linken als auch in der rechten Niere zertrümmert werden können. Die Schwenkbewegung des Stoßwellenerzeugers wird dabei durch eine Schwenkung des an dem unterhalb der Tischplatte befindlichen Lagerhalter angelenkten Schwenkarms bewirkt. In weiterer Ausgestaltung der Erfindung ist vorgesehen, daß der Lagerhalter für den Schwenkarm um eine dritte Achse schwenkbar ist, die unter dem Schwenkarm verläuft. Diese Ausgestaltung ermöglicht es, durch eine Schwenkung um die zweite und die dritte Achse den Stoßwellenerzeuger in eine Parkstellung zu schwenken, in der er eine Röntgenuntersuchung mit der Röntgeneinrichtung nicht behindert. Er kann aber auch in einer Servicestellung geschwenkt werden, in der der Stoßwellenerzeuger besonders leicht für den Service zugänglich ist.

Wenn ein Konkrement in dem Patienten geortet und in den Schnittpunkt der ersten Achse und des Zentralstrahls gerückt worden ist, muß der Lagerhalter lediglich in eine definierte Stellung geschwenkt werden in der der Stoßwellenerzeuger – bei jeder beliebigen Winkelstellung in bezug auf die zweite Achse – auf das Konkrement fokussiert ist. Eine solche Ausrichtung des Stoßwellenerzeugers ist wesentlich präziser als bei dem eingangs erwähnten Arbeitsplatz, bei dem der Wagen, der den c-förmigen Träger für den Stoßwellenerzeuger trägt, auf dem Boden in eine exakt definierte Stellung geschoben werden muß.

In weiterer Ausgestaltung der Erfindung ist vorgesehen, daß das Lager für den Schwenkarm um eine dritte Achse schwenkbar ist, die unter dem Schwenkarm, vorzugsweise parallel zur Tischlängsrichtung, verläuft. Diese Ausgestaltung ermöglicht es, durch eine Schwenkung um die zweite und die dritte Achse den Stoßwellenerzeuger in eine Parkstellung zu schwenken, in der er eine Röntgenuntersuchung mit der Röntgeneinrichtung nicht behindert. Er kann aber auch in eine Servicestellung geschwenkt werden, in der der Stoßwellenerzeuger besonders leicht für den Service zugänglich ist.

In weiterer Ausgestaltung der Erfindung ist vorgesehen, daß der Bildwandler und/oder der Röntgenstrahler an einem zur ersten Achse senkrechten Arm um dessen Längsachse schwenkbar ist. Dies erlaubt es, den Strahlengang umzukehren, indem zunächst der an dem Arm schwenkbar befestigte Teil um etwa 90° gegenüber der Untersuchungsstellung geschwenkt, dann die Röntgeneinrichtung um die erste Achse um 180° geschwenkt und schließlich der erwähnte Teil wieder in die Untersuchungsstellung zurückgeschwenkt wird.

Eine weitere Ausgestaltung der Erfindung, mit der auch Röntgenuntersuchungen mit einem schrägen Strahleneinfall in einer zur Tischlängsrichtung senkrechten Ebene möglich sind, ist dadurch gekennzeichnet, daß der Patientenlagerungstisch unabhängig von der Röntgeneinrichtung um eine durch den Schnittpunkt verlaufende vertikale Achse schwenkbar ist.

Durch das Schwenken des Patientenlagerungstisches um die vertikale Achse können Röntgenaufnahmen mit weitgehend beliebiger Strahleneinfallsrichtung in bezug auf den Patienten angefertigt werden.

In Weiterbildung der Erfindung ist vorgesehen, daß der Schwenkarm für den Stoßwellenerzeuger mit dem Patientenlagerungstisch verbunden ist. Dadurch ist es möglich, eine Lithotripsie in jeder beliebigen Schwenkstellung des Patientenlagerungstisches, d.h. ohne diesen in diesen Ausgangslage zurückzuschwenken, vorzunehmen.

Die Erfindung wird nachstehend anhand der Zeichnung näher erläutert. Es zeigen:

Fig. 1 eine Seitenansicht eines Arbeitsplatzes nach der Erfindung,

Fig. 2 eine Frontansicht eines solchen Arbeitsplatzes bei geänderter Stellung des Stoßwellenerzeugers,

Fig. 3 einen Ausschnitt aus dieser Ansicht mit verschiedenen Stellungen des Stoßwellenerzeugers, und

Fig. 4 eine Draufsicht auf einen solchen Arbeitsplatz.

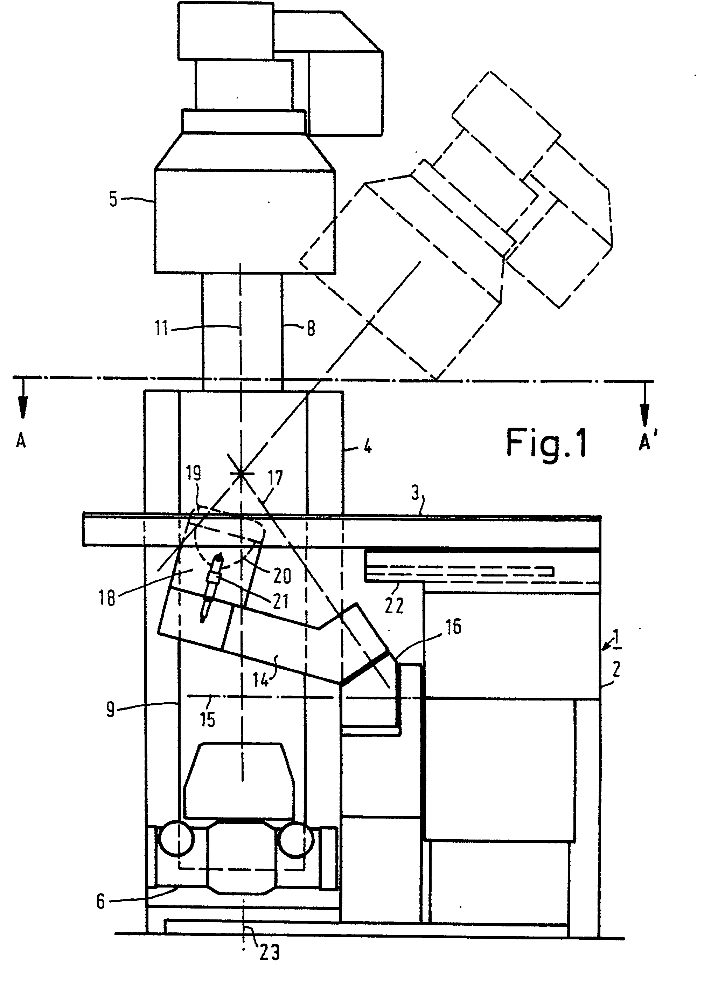

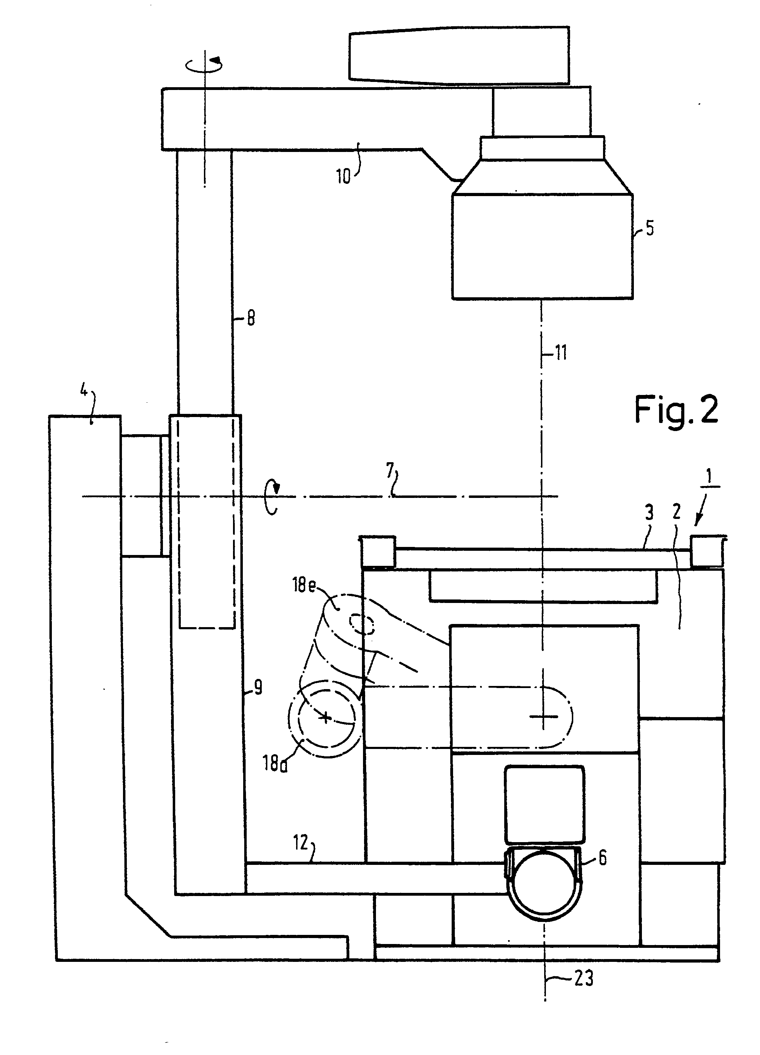

In den Fig. 1 und 2 ist mit 1 ein Patientenlagerungstisch mit einem Sockel 2 und einer Tischplatte 3 bezeichnet, die in ihrer Längsrichtung (in Fig. 1 horizontal), in Querrichtung (senkrecht zur Zeichenebene der Fig. 1) und in der Höhe verstellbar ist. An einem Stativ 4 ist eine Röntgeneinrichtung um eine horizontale, zur Tischlängsrichtung senkrechte Achse 7 schwenkbar angeordnet. Die Röntgeneinrichtung umfaßt einen Röntgenstrahler 6, der an einem Träger 12 am Ende eines um die Achse 7 schwenkbaren Hohlarmes 9 befestigt ist, und einen Röntgenbildverstärker 5. Der Röntgenbildverstärker ist an einem Träger 10 befestigt, der seinerseits am Ende eines Armes 8 um dessen Längsachse schwenkbar befestigt ist. Der Arm 8 kann innerhalb des Hohlarms 9 verschoben werden, so daß der Abstand zwischen dem Röntgenbildverstärker 5 und dem Röntgenstrahler 6 geändert werden kann.

Unter der Tischplatte befindet sich ein um eine horizontale Achse 15 schwenkbarer Lagerhalter 16, in dem ein Schwenkarm 14 um eine Achse 17 schwenkbar gelagert ist, die mit der Achse 15 einen Winkel von ca. 55° einschließt. Am freien Ende des Schwenkarms 14 ist ein Stoßwellengenerator 18 befestigt. Der Stoßwellengenerator enthält einen mit Wasser gefüllten, durch eine Membran 19 abgeschlossenen Hohlraum in Form eines Rotationsellipsoids 20, in dessen einem Brennpunkt sich eine Funkenstrecke 21 zur Erzeugung einer Stoßwelle befindet. Der andere Brennpunkt des Rotationsellipsoids – im folgenden Fokuspunkt genannt – liegt auf der Schwenkachse 17. In ihm konzentriert sich in bekannter Weise die durch die Stoßwelle erzeugte Energie. Die Energie zur Steinzertrümmerung kann auch auf andere Weise erzeugt werden, beispielsweise dadurch, daß auf einem geeignet geformten Rotationskörper Ultraschallwandler angeordnet sind, deren Energie in einem Punkt fokussiert wird.

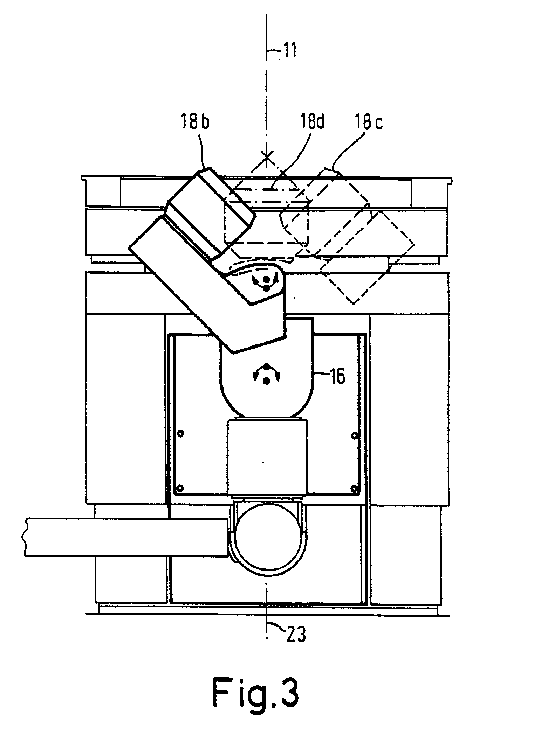

Wenn der Lagerhalter sich in der Position (vgl. Fig. 1 und Fig. 3) befindet, in der die Schwenkachse 17 mit der Schwenkachse 15 eine vertikale, zur Tischlängsrichtung parallele Ebene definiert, fällt der Fokuspunkt mit dem Schnittpunkt des Zentralstrahls und der horizontalen Schwenkachse 7 zusammen. Wenn der Patient zuvor so positioniert ist, daß der zu zertrümmernde Stein sich in diesem Schnittpunkt befindet, kann in dieser Stellung des Lagerhalters der Schwenkarm 14 so um die Achse 17 geschwenkt werden, bis eine im Hinblick auf die Anatomie des Patienten optimale Position erreicht ist, ohne daß dadurch die Fokussierung beeinträchtigt wird. Der beschriebene Arbeitsplatz ist nicht nur in Verbindung mit der Lithotripsie verwendbar, sondern auch für urologische oder andere Röntgenuntersuchungen - wie im folgenden erläutert wird.

Zur Anfertigung von Untertischaufnahmen wird der Stoßwellenerzeuger 18 aus dem Strahlengang geschwenkt. Dies kann beispielsweise dadurch erfolgen, daß der Lagerhalter 16 um die Achse 15 so geschwenkt wird, daß die Achsen 15 und 17 eine horizontale Ebene definieren und daß der Schwenkarm um die Achse 17 um 180° geschwenkt wird. Er nimmt dann die in Fig. 2 gestrichelt angedeutete und mit 18a bezeichnete Parkstellung ein.

Es ist aber auch möglich, Röntgenuntersuchungen mit umgekehrten Strahlengang durchzuführen. Zu diesem Zweck wird zunächst der Arm 8 ganz in den Hohlarm 9 hineingefahren, der Träger 10 um den Arm 8 geschwenkt und dann die Röntgeneinrichtung um 180° um die Achse 7 geschwenkt, wobei sich der Röntgenstrahler um das Fußende herum nach oben und der Bildverstärker in dem Raum zwischen dem Tisch 1 und dem Stativ 4 nach unten bewegt. Dort wird der Träger 10 wieder zurückgeschwenkt, so daß der Röntgenstrahler 6 und der Bildverstärker 5 wieder aufeinander zentriert sind. In dieser Stellung der Röntgenröhre können auch Buckyaufnahmen mit Hilfe einer unter der Tischplatte 3 angeordneten, in Tischlängsrichtung verfahrbaren Kassettenlade 22 angefertigt werden.

Zur Ortung von Konkrementen im menschlichen Körper, vorzugsweise von Nierensteinen, wird der Patient zunächst in der in Fig. 1 mit ausgezogenen Linien dargestellten Stellung der Röntgeneinrichtung durchstrahlt und nach einer Schwenkung der Röntgeneinrichtung um die Achse 7 (die in Fig. 1 für den Bildwandler 5 gestrichelt angedeutet ist) erneut aus der so veränderten Perspektive. Dies macht es möglich, die räumliche Lage des zu zertrümmernden Steins zu bestimmen und die Tischplatte so zu verschieben, daß sich der Stein im Schnittpunkt der horizontalen Achse 7 mit dem Zentralstrahl 11 befindet.

Danach wird der Lagerhalter 16 so geschwenkt, daß die Schwenkachsen 15 und 17 wieder eine vertikale Ebene definieren. In dieser Stellung des Lagerhalters 16 ist der Stoßwellenerzeuger 18 unabhängig von der Stellung des Schwenkarms 14 stets auf den Schnittpunkt der Schwenkachse 7 mit dem Zentralstrahl 11 fokussiert, und er bleibt es auch, wenn der Schwenkarm in die für die Steinzertrümmerung optimale Position geschwenkt wird.

Bei der Zertrümmerung eines Steins in der rechten Niere wird der Stoßwellenerzeuger - wie in Fig. 3 mit ausgezogenen Linien angedeutet und mit 18b bezeichnet - nach links geschwenkt, bis die Wirkungslinie des Stoßwellenerzeugers zusammen mit der horizontalen Schwenkachse zumindest annähernd eine vertikale, zur Tischlängsrichtung senkrechte Ebene definieren. Die Wirkungsrichtung verläuft dann etwa unter einem Winkel von 43° in bezug auf die Horizontale. Bei der Zertrümmerung eines Steins in der linken Niere wird der Stoßwellenerzeuger analog nach rechts geschwenkt (gestrichelte Position 18c) und bei einer Untersuchung eines Gallensteins in eine mittlere Position (18d bzw. Fig. 1).

In all diesen Fällen muß die Membran 19 des Stoßwellenerzeugers 18 unmittelbaren Kontakt mit dem Körper des Patienten haben; dementsprechend muß eine nicht näher dargestellte Öffnung in der Tischplatte 3 vorhanden sein.

Bei einem Stoßwellenerzeuger, bei dem die Stoßwelle mittels einer Funkenstrecke erzeugt wird, muß in regelmäßigen Abständen ein Elektrodenwechsel vorgenommen werden. Zu diesem Zweck wird aus der in Fig. 1 bzw. Fig. 3 (Postion 18d) dargestellten Position der Schwenkarm 14 um 90° um die Achse 17 und der Lagerhalter 16 um 90° um die Achse 15 geschwenkt, so daß der Stoßwellenerzeuger die in Fig. 2 mit 18e bezeichnete Position einnimmt, bei der die Öffnung des Rotationsellipsoids 20 im Stoßwellenerzeuger 18 nach unten gerichtet ist. In dieser Stellung kann die Funkenstrecken-Elektrode 21 entnommen werden, ohne daß die Wasserfüllung in dem durch den Rotationselipsoid 20 und die Membran 19 abgebildeten Raum verloren geht.

Bei dem bisher beschriebenen Gerät ist es lediglich möglich, den auf den Patientenlagerungstisch 1 liegenden Patienten mit einer solchen Durchstrahlungsrichtung zu durchstrahlen, daß der Zentralstrahl stets in einer die Tischlängsachse enthaltenden vertikalen Ebene verbleibt.

Der Benutzer kann dabei nur von einer Seite (in Fig. 2 von rechts) an den Patienten herantreten. In vielen Fällen ist es aber erwünscht, daß der Patient beidseitig zugänglich gelagert ist bzw. daß der Patient aus anderen Richtungen durchstrahlt werden kann.

Zu diesem Zweck ist der Patientenlagerungstisch mitsamt dem damit über die Teile 16, 14 verbundenen Stoßwellenerzeuger 18 um eine vertikale Achse 23 schwenkbar, die durch den Fokuspunkt verläuft und damit in der in Fig. 1 und 2 dargestellten Stellung der Röntgeneinrichtung 5, 6 mit dem Zentralstrahl 11 zusammenfällt. Zu diesem Zweck kann das in den Zeichnungen nur schematisch dargestellte Fußgestell des Sockels 2 des Patientenlagerungstisches 1 in einem nicht näher dargestellten, in den Fußboden eingelassenen zentralen Lager oder Drehkranz gelagert sein. Am Sockel können darüber hinaus ebenfalls nicht näher dargestellte, im Abstand von der Achse 23 angeordnete Rollen vorgesehen sein, die einen Teil des Gewichts des Tisches 1 abfangen und die auf dem Fußboden bzw. einer darin versenkten Schienenanordnung abrollen.

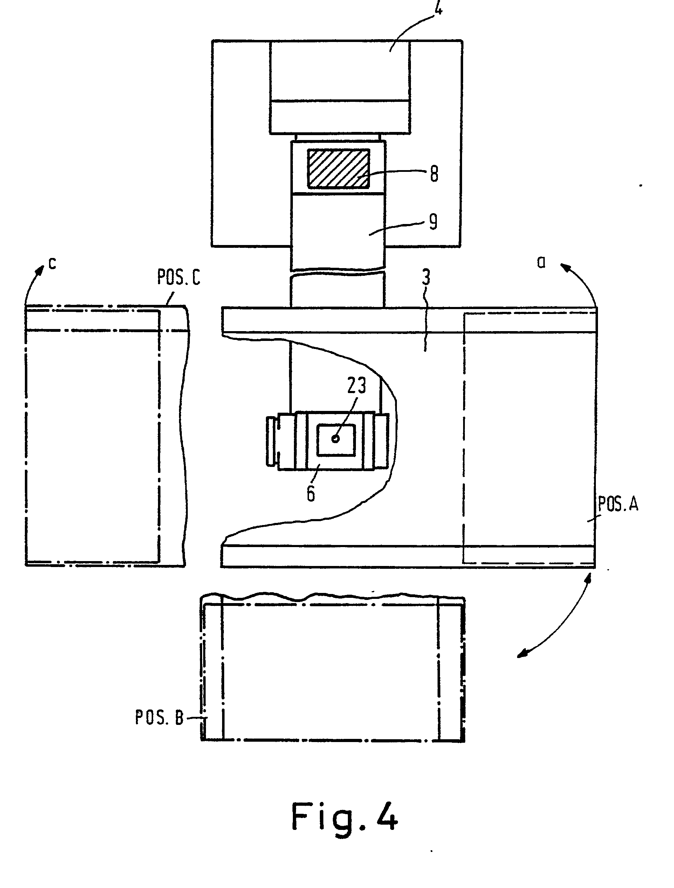

Fig. 4, die eine Draufsicht auf das Gerät entsprechend der Linie A, A' in Fig. 1 darstellt (wobei die Tischplatte 3 teilweise aufgebrochen gezeichnet und der Stoßwellenerzeuger mitsamt den Teilen 14 und 16 weggelassen ist), zeigt die damit möglichen Untersuchungspositionen. Die Position A ist die in Fig. 1 dargestellte Position.

Durch Schwenken des Patientenlagerungstisches um 90° um die horizontale Achse 23 wird die Position B erreicht. Diese Position ist insofern günstig, als der Patient dann von beiden Seiten zugänglich ist. In dieser Position kann der durch die Arme 8, 9 und die Träger 10, 12 gebildete Bogen um die Achse 7 unmittelbar um 180° geschwenkt werden, so daß der Patient aus beliebigen Richtungen mit einem in einer zur Tischlängsachse senkrechten Ebene liegenden Zentralstrahl 11 durchstrahlt werden kann.

Der Patientenlagerungstisch 10 kann darüber hinaus in die Position C verfahren werden, in der die andere Seite des Patienten (im Vergleich zu Position A) für den Untersucher zugänglich ist. In sämtlichen Positionen kann die Tischplatte 1 noch über das Kopfende hinaus verschoben werden. Außerdem ist es möglich, in den Positionen A und C den Patientenlagerungstisch noch um 45° im Gegenuhrzeigersinn bzw. im Uhrzeigersinn zu verschwenken, wie durch die Pfeile a und c angedeutet, so daß sich insgesamt ein Schwenkbereich von 270° ergibt.

**Patentansprüche**

1. Lithotripsie-Arbeitsplatz mit einem Patientenlagerungstisch (1; 2, 3), einer einen Röntgenstrahler (6) und einen darauf zentrierten Bildwandler (5) umfassenden Röntgeneinrichtung zur Ortung von Konkrementen, die um eine erste zur Tischlängsrichtung senkrechte, horizontale Achse (7) schwenkbar ist, die den Zentralstrahl der Röntgeneinrichtung (11) in einem Schnittpunkt schneidet, und mit einem unterhalb der Tischplatte (3) befindlichen Stoßwellenerzeuger (18), der um eine durch seinen Fokuspunkt verlaufende zweite Achse bewegbar ist, wobei die Röntgeneinrichtung (5, 6) und der Stoßwellenerzeuger (18) relativ zueinander so anordbar sind, daß der Fokuspunkt und der Schnittpunkt zusammenfallen, dadurch gekennzeichnet, daß die zweite Achse (17) schräg verläuft und mit der Tischlängsrichtung eine vertikale Ebene definiert und daß der Stoßwellenerzeuger (18) an einem Schwenkarm (14) befestigt ist, der an einem unterhalb der Tischplatte (3) befindlichen Lagerhalter (16) um die zweite Achse (17) schwenkbar angelenkt ist.

2. Arbeitsplatz nach Anspruch 1, dadurch gekennzeichnet, daß der Lagerhalter für den Schwenkarm (14) um eine dritte Achse (15) schwenkbar ist, die unter dem Schwenkarm verläuft.

3. Arbeitsplatz nach Anspruch 1 oder 2, dadurch gekennzeichnet, daß der Bildwandler und/oder der Röntgenstrahler an einem zur ersten Achse (7) senkrechten Arm (8) und dessen Längsachse schwenkbar ist.

4. Arbeitsplatz nach Anspruch 3, dadurch gekennzeichnet, daß der Arm (8) in einem um die erste Achse (7) schwenkbaren Hohlarm (9) in seiner Längsrichtung verschiebbar angeordnet ist.

5. Arbeitsplatz nach einem der Ansprüche 1 bis 4, dadurch gekennzeichnet, daß der Patientenlagerungstisch (1) unabhängig von der Röntgeneinrichtung (4,5,6) um eine durch den Schnittpunkt verlaufende vertikale Achse (23) schwenkbar ist.

6. Arbeitsplatz nach Anspruch 5,
<u>dadurch gekennzeichnet</u>, daß der Schwenkarm (14) für den Stoßwellenerzeuger (18) mit dem Patientenlagerungstisch verbunden ist.

**Claims**

1. An operation site for lithotripsy, comprising a patient table (1, 2, 3), an X-ray device which comprises an X-ray source (6) and an image converter (5), centered with respect thereto, and which serves to localize concrements, which X-ray device is pivotable about a first horizontal axis (7) which extends perpendicularly to the longitudinal direction of the table and which intersects the central ray (11) of the X-ray device in a point of intersection, and also comprising a shock wave generator (18) which is arranged underneath the table top (3) and which is displaceable about a second axis which extends through its focal point, it being possible to arrange the X-ray device (5, 6) and the shock wave generator (18) with respect to one another so that the focal point and the point of intersection coincide, characterized in that the second axis (17) is inclined and defines a vertical plane with the longitudinal direction of the table, the shock wave generator (18) being connected to a pivot arm (14) which is journalled to a bearing holder (16), arranged underneath the table top (3), so as to be pivotable about the second axis (17).

2. An operation site as claimed in Claim 1, characterized in that the bearing holder for the pivot arm (14) is pivotable about a third axis (15) which extends underneath the pivot arm.

3. An operation site as claimed in Claim 1 or 2, characterized in that the image converter and/or the X-ray source are connected to an arm (8) which is perpendicular to the first axis (7) in order to be pivotable about the longitudinal axis of said arm.

4. An operation site as claimed in Claim 3, characterized in that the arm (8) is arranged so as to be slidable in its longitudinal direction in a hollow arm (9) which is pivotable about the first axis (7).

5. An operation site as claimed in any one of the Claims 1 to 4, characterized in that the patient table (1) is pivotable, independently of the X-ray device (4, 5, 6), about a vertical axis (23) which extends through the point of intersection.

6. An operation site as claimed in Claim 5, characterized in that the pivot arm (14) for the shock wave generator (18) is connected to the patient table.

**Revendications**

1. Site opératoire pour lithotripsie comportant une table pour le patient (1; 2, 3), un appareil à rayons X destiné à localiser des concrétions qui comprend un générateur de rayons X (6) et un convertisseur d'image (5) centré sur celui-ci et peut pivoter autour d'un premier axe horizontal (7) perpendiculaire à la direction longitudinale de la table qui coupe le rayon central (11) de l'appareil à rayons X en un point d'intersection, ainsi qu'un générateur d'ondes de choc (18) situé en dessous du plateau (3) de la table qui est mobile autour d'un deuxième axe passant par son point focal, l'appareil à rayons X et le générateur d'ondes de choc pouvant être disposés l'un par rapport à l'autre d'une manière telle que le point focal et le point d'intersection coïncident, caractérisé en ce que le deuxième axe (17) présente une obliquité par rapport au premier et définit un plan vertical avec la direction longitudinale de la table et que le générateur d'ondes de choc (18) est fixé à un bras pivotant (14) qui est articulé à un bloc-palier (16) situé en dessous du plateau (3) de la table de manière à pouvoir pivoter autour du deuxième axe (17).

2. Site opératoire suivant la revendication 1, caractérisé en ce que le bloc-palier pour le bras pivotant (14) peut pivoter autour d'un troisième axe (15) qui s'étend en dessous du bras pivotant.

3. Site opératoire suivant la revendication 1 ou 2, caractérisé en ce que le convertisseur d'image et/ou le générateur de rayons X sont fixés sur un bras (8) perpendiculaire au premier axe (7), de manière à pouvoir pivoter autour de l'axe longitudinal de ce bras.

4. Site opératoire suivant la revendication 3, caractérisé en ce que le bras (8) est monté de manière à pouvoir coulisser dans le sens de sa longueur dans un bras creux (9) qui peut pivoter autour du premier axe (7).

5. Site opératoire suivant l'une quelconque des revendications 1 à 4, caractérisé en ce que la table (1) pour le patient peut pivoter indépendamment de l'appareil à rayons X (4, 5, 6) autour d'un axe vertical (23) passant par le point d'intersection.

6. Site opératoire suivant la revendication 5, caractérisé en ce que le bras pivotant (14) pour le générateur d'ondes de choc (18) est relié à la table pour le patient.

Fig.1

Fig.2

Fig.3

Fig.4